# EUROPEAN PATENT SPECIFICATION

(11) **EP 3 831 422 B1**
(45) Date of publication and mention of the grant of the patent: **21.09.2022**
(21) Application number: 19861576.7
(22) Date of filing: 08.07.2019
(51) Int. Cl.: A61M 1/28, A61M 1/16, A61P 7/08, B01D 61/24

(54) **MANUFACTURING DEVICE FOR HYDROGEN-INCLUDING PERITONEAL DIALYSIS FLUID**
VORRICHTUNG ZUR HERSTELLUNG EINER WASSERSTOFFHALTIGEN PERITONEALDIALYSEFLÜSSIGKEIT
DISPOSITIF DE FABRICATION D'UN FLUIDE DE DIALYSE PÉRITONÉALE COMPRENANT DE L'HYDROGÈNE

(30) Priority: 21.09.2018 JP 2018176943
(43) Date of publication of application: 09.06.2021
(73) Proprietor: Nihon Trim Co., Ltd., Osaka 531-0076 (JP)
(72) Inventor: TACHIBANA Takashi, Nankoku-shi, Kochi 783-0060 (JP)
(74) Representative: Germain Maureau
(86) International application number: PCT/JP2019/027051
(87) International publication number: WO 2020/059259

(56) References cited:
- JP-A- 2009 125 654
- JP-A- 2014 161 605
- JP-A- 2016 077 675
- JP-A- 2016 077 675
- NAKAYAMA M. ET AL: "A novel bioactive haemodialysis system using dissolved dihydrogen (H2) produced by water electrolysis: a clinical trial", NEPHROLOGY DIALYSIS TRANSPLANTATION, vol. 25, no. 9, 12 April 2010 (2010-04-12) , pages 3026-3033, XP055830633, GB ISSN: 0931-0509, DOI: 10.1093/ndt/gfq196

## Description

### TECHNICAL FIELD

The present invention relates to an apparatus for manufacturing hydrogen-containing peritoneal dialysate.

### BACKGROUND ART

Peritoneal dialysis has been known as one of the effective treatments for patients with renal failure who have decreased renal function and are unable to excrete urine to regulate water content and remove harmful substances inside the body including waste products such as urea.

The peritoneal dialysis is a method of removing waste products and water existing in the capillaries of the patient's peritoneum by injecting dialysate into the patient's peritoneal cavity. In the peritoneal dialysis, peritoneal dialysate having a higher osmotic pressure than body fluid (blood) is infused, and waste products and water are removed from the patient by utilizing a difference in the osmotic pressure between the body fluid and the peritoneal dialysate through the peritoneum.

Further, in recent years, it has been observed that oxidative stress occurs in dialysis patients in the peritoneal dialysis. It is considered that the oxidative stress is caused by active oxygen generated during dialysis, and it has been proposed to eliminate this active oxygen to reduce the oxidative stress.

For example, a method has been proposed according to which dialysate having a high concentration of hydrogen dissolved therein is produced by adding hydrogen molecules in dialysate enclosed in a container. Specifically, a method of adding hydrogen molecules in dialysate without opening a container is disclosed. According to the method, hydrogen molecules are brought into contact with a container (dialysate bag) having dialysate enclosed therein and having hydrogen molecule permeability from the outside of the container (the container is immersed in hydrogen-containing water, or the container is brought into contact with hydrogen gas). It is disclosed that such a method can avoid the problem of deterioration of dialysate quality due to opening of the container (see, for example, Patent Document 1).

### CITATION LIST

### PATENT DOCUMENTS

Patent Document 1: Japanese Patent No. 4486157

JP 2016 077675 A discloses a peritoneal dialysis fluid manufacturing device including: a container having hydrogen permeability with a pre-set capacity; dialysis fluid accommodated in the container; a hydrogen water storage tank in which the container in which the dialysis fluid is accommodated is immersed for storing hydrogen water containing hydrogen supplied to the dialysis fluid; a hydrogen water generation device connected to the hydrogen water storage tank for generating the hydrogen water; and a circulation passage for circulating the hydrogen water between the hydrogen water storage tank and the hydrogen water generation device.

JP 2014 161605 A relates to a hydrogen addition device including: a flow passage formed of a gas permeable membrane which selectively allows hydrogen to permeate for delivering a liquid; and a hydrogen addition part for enclosing a part of the flow passage with a hydrogen transporting fluid, and adding hydrogen to the liquid by making the hydrogen contained in the hydrogen transporting fluid permeate through the gas permeable membrane.

### SUMMARY OF THE INVENTION

### TECHNICAL PROBLEM

However, the method of producing peritoneal dialysate disclosed in Patent Document 1 involves a problem in that the container needs to be taken out of the hydrogen-containing water for dialysis after the container with the dialysate enclosed therein is immersed in the hydrogen-containing water, resulting in decreased convenience.

Further, the hydrogen-containing water adheres to the outer surface of the container because the container with the dialysate enclosed therein is immersed in the hydrogen-containing water. As a result, there arises a problem that various bacteria and the like grow on the outer surface of the container with the dialysate enclosed therein, resulting in that dialysate is subject to contamination during peritoneal dialysis.

Therefore, the present invention has been made in view of the above problems, and an object of the present invention is to provide an apparatus for manufacturing hydrogen-containing peritoneal dialysate, which is excellent in terms of convenience and hygiene.

### SOLUTION TO THE PROBLEM

In order to achieve the above objective, the apparatus for manufacturing hydrogen-containing peritoneal dialysate of the present invention includes: a dialysate bag housing dialysate to be injected into a peritoneal cavity of a patient; and a hydrogen dissolution device interposed between the dialysate bag and the patient and configured to dissolve hydrogen in the dialysate, wherein the hydrogen dissolution device includes: an air supply module connected to the dialysate bag and the patient and configured to dissolve hydrogen in the dialysate; and a hydrogen supply device connected to the air supply module and configured to supply hydrogen to the air supply module.

### ADVANTAGES OF THE INVENTION

The present invention makes it possible to obtain peritoneal dialysate having a desired dissolved hydrogen concentration while performing peritoneal dialysis, thereby improving convenience of the peritoneal dialysis. In addition, hydrogen-containing peritoneal dialysate excellent in terms of hygiene can be obtained.

### BRIEF DESCRIPTION OF THE DRAWINGS

FIG. 1 is a schematic diagram of a configuration of an apparatus for manufacturing hydrogen-containing peritoneal dialysate according to an embodiment of the present invention.
FIG. 2 is a perspective view for explaining the air supply module in the apparatus for manufacturing hydrogen-containing peritoneal dialysate according to the embodiment of the present invention.
FIG. 3 is an exploded perspective view for explaining the air supply module in the apparatus for manufacturing hydrogen-containing peritoneal dialysate (not claimed).
FIG. 4 is a diagram of a configuration of a peritoneal dialysis apparatus according to the embodiment of the present invention.
FIG. 5 is an exploded perspective view for explaining the air supply module according to the present invention.
FIG. 6 is a schematic diagram of a configuration of an apparatus for manufacturing hydrogen-containing peritoneal dialysate according to a variation of the present invention.

### DESCRIPTION OF EMBODIMENTS

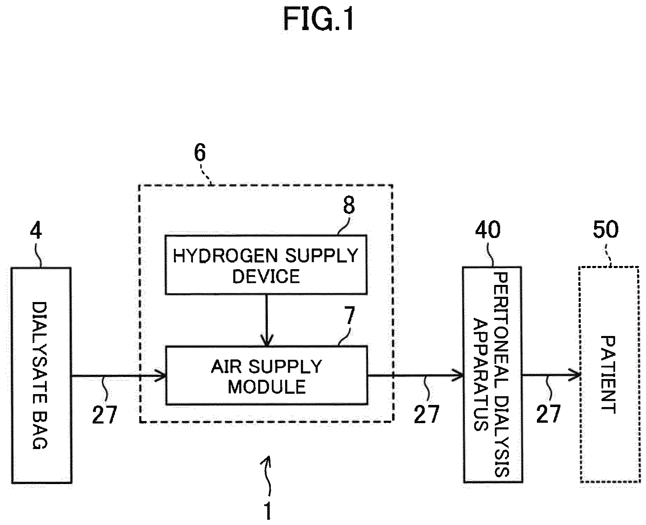

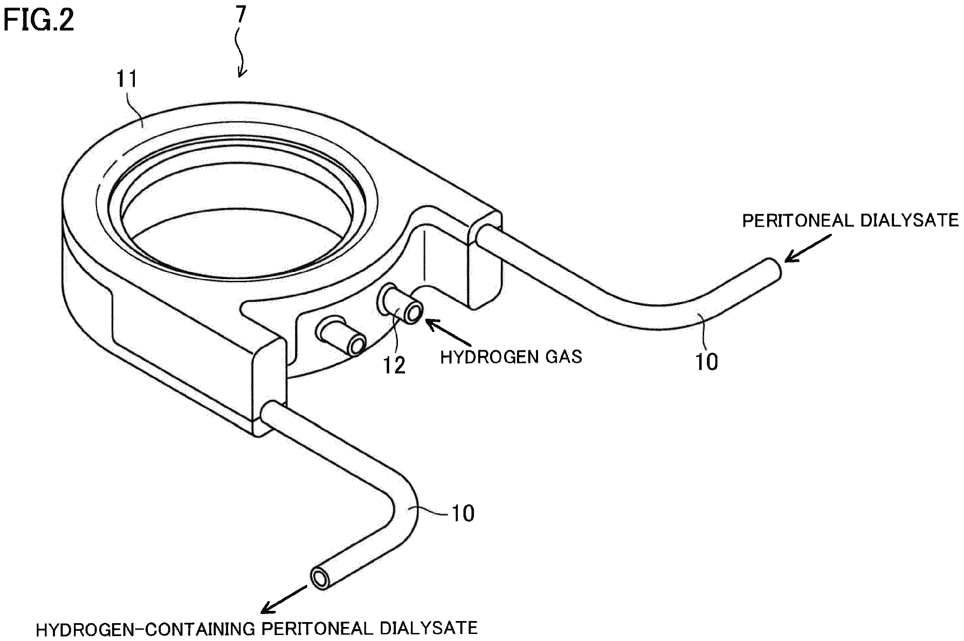

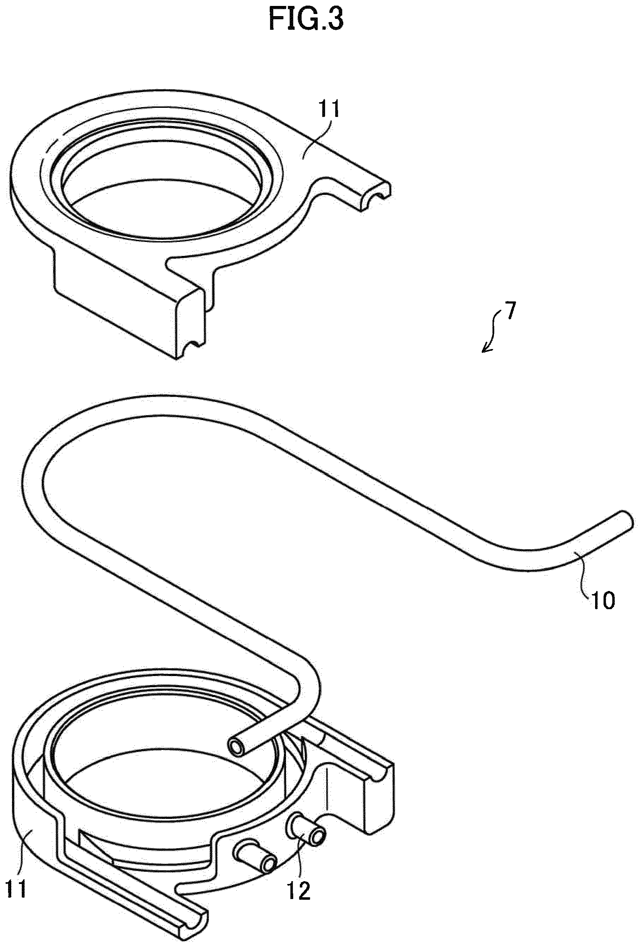

FIG. 1 is a conceptual diagram showing a configuration of an apparatus for manufacturing hydrogen-containing peritoneal dialysate according to an embodiment of the present invention. FIGS. 2 and 3 are perspective views for explaining an air supply module in the apparatus for manufacturing hydrogen-containing peritoneal dialysate (figure 3: not claimed).

The apparatus 1 for manufacturing hydrogen-containing peritoneal dialysate includes a dialysate bag 4 housing dialysate 27, a peritoneal dialysis apparatus 40 connected to the dialysate bag 4 and supplied with the dialysate 27 from the dialysate bag 4, and a hydrogen dissolution device 6 interposed between the dialysate bag 4 and the peritoneal dialysis apparatus 40 and dissolving hydrogen in the dialysate 27.

The hydrogen dissolution device 6 includes an air supply module 7 connected to the dialysate bag 4 and the peritoneal dialysis apparatus 40 and dissolving hydrogen in the dialysate 27 and a hydrogen supply device 8 connected to the air supply module 7 and supplying hydrogen to the air supply module 7.

As shown in FIGS. 2 and 3, the air supply module 7 has a supply tube 10 connected to the dialysate bag 4 and the peritoneal dialysis apparatus 40 and supplying the dialysate 27 to the peritoneal dialysis apparatus 40, and an air supply module body 11 connected to the hydrogen supply device 8, housing the supply tube 10, and supplied with hydrogen.

The supply tube 10 has hydrogen molecule permeability, and the dialysate 27 housed in the dialysate bag 4 is supplied to the inside of the supply tube 10. A material forming the supply tube 10 is not limited as long as the material allows hydrogen gas to permeate, and for example, a material having hydrogen permeability such as polyethylene, polypropylene, or polystyrene can be used.

Further, the air supply module body 11 is provided with a hydrogen supply port 12, and hydrogen gas is supplied to the outside of the supply tube 10 by the hydrogen supply device 8 through the hydrogen supply port 12.

Then, with the dialysate 27 supplied to the inside of the supply tube 10, hydrogen is supplied to the dialysate 27 by passing through the inside of the air supply module body 11 outside of the supply tube 10.

The hydrogen supply device 8 is not particularly limited as long as it can supply hydrogen to the air supply module 7. For example, a hydrogen generator that electrolyzes water, a hydrogen generating agent such as magnesium hydride that reacts with water to generate hydrogen, a hydrogen gas cylinder, and the like can be used.

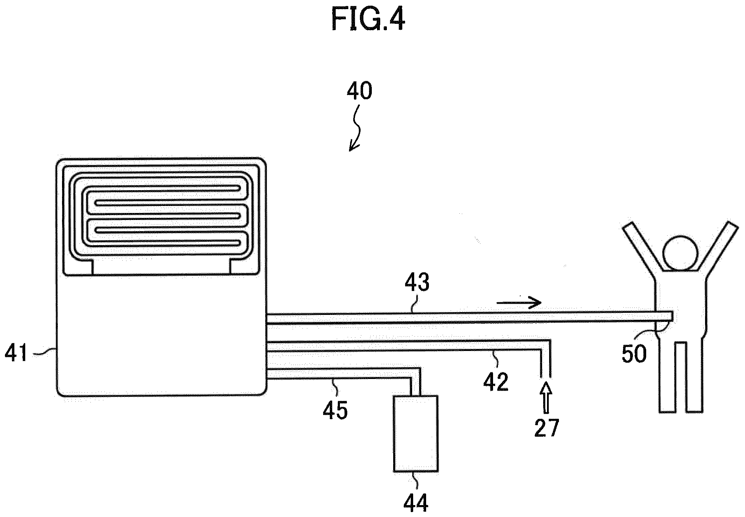

As shown in FIG. 4, the peritoneal dialysis apparatus 40 includes a dialysis apparatus body 41, a supply tube 42 into which the manufactured dialysate 27 is introduced and supplies the dialysate 27 to the dialysis apparatus body 41, and a dialysis catheter 43 connected to the dialysis apparatus body 41 and the peritoneal cavity of a patient 50 and supplying the dialysate 27 to the patient 50. Further, the peritoneal dialysis apparatus 40 includes a waste fluid treatment tank 44 for discarding unused dialysate 27, and a connecting tube 45 connecting the dialysis apparatus body 41 and the waste fluid treatment tank 44.

The dialysate 27 is supplied to the dialysis apparatus body 41 via the supply tube 42. Then, the dialysate 27 is injected into the peritoneal cavity of the patient 50 via the dialysis catheter 43 by the dialysis apparatus body 41, which is an injection treatment, and the dialysate 27 is drained from the peritoneal cavity, which is a drainage treatment.

Then, as shown in FIG. 1, the dialysate 27 supplied with hydrogen is supplied to the peritoneal dialysis apparatus 40, the dialysate 27 thus supplied is supplied from the peritoneal dialysis apparatus 40 to the patient 50, and thus the blood of the patient 50 is purified.

As described above, in the present embodiment, the hydrogen dissolution device 6 dissolving hydrogen in the dialysate 27 is provided between the dialysate bag 4 and the peritoneal dialysis apparatus 40.

This makes it possible to obtain the dialysate 27 including hydrogen while performing peritoneal dialysis, thereby improving convenience of the peritoneal dialysis. In addition, the dialysate 27 excellent in terms of hygiene can be obtained.

The hydrogen dissolution device 6 only includes the air supply module 7 dissolving hydrogen in the dialysate 27 and the hydrogen supply device 8 supplying hydrogen to the air supply module 7, and thus can supply hydrogen to the dialysate 27 with a simple configuration.

Further, the air supply module 7 having the supply tube 10 is used to bring hydrogen into contact with the dialysate 27 to dissolve the hydrogen, and thus hydrogen can be efficiently supplied.

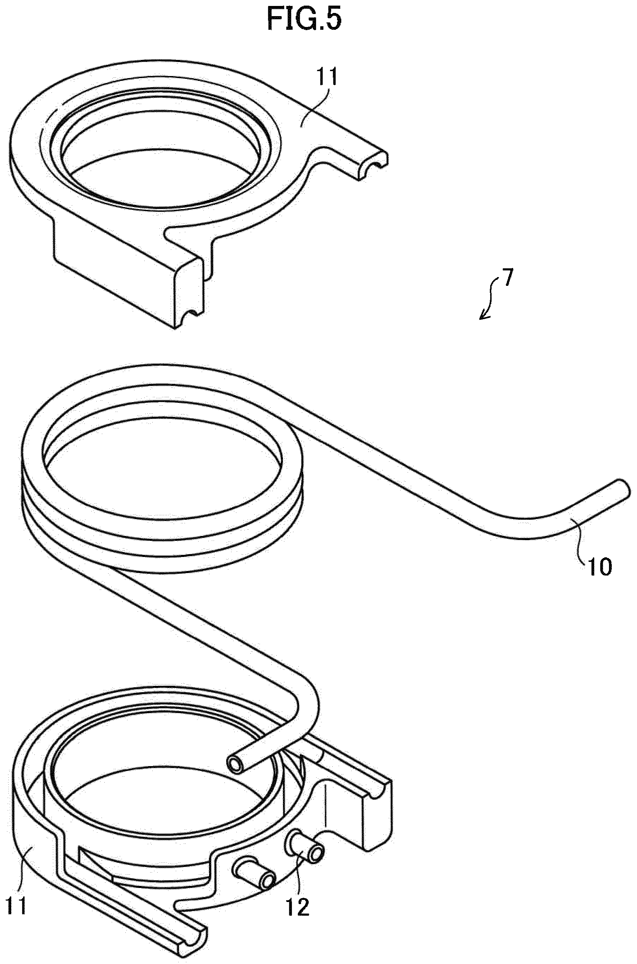

As shown in FIG. 5, in the air supply module 7, the supply tube 10 is wound in a roll shape and housed in the air supply module body 11. This configuration increases a contact area between the supply tube 10 and hydrogen and allows hydrogen to be supplied to the dialysate 27 even more efficiently.

Further, instead of the air supply module 7 including the supply tube 10 and the air supply module body 11 described above, an air supply module having a plurality of hollow fibers connected to the dialysate bag 4 and the peritoneal dialysis apparatus 40 may be used.

In this case, the dialysate 27 housed in the dialysate bag 4 is supplied to the inside of the hollow fibers, and hydrogen is supplied to the outside of the hollow fibers by the hydrogen supply device 8.

Then, hydrogen is supplied to the dialysate 27 by passing hydrogen through the outside of the hollow fibers with dialysate 27 supplied to the inside of the hollow fibers.

In one not claimed embodiment, the air supply module 7 including the supply tube 10 and the air supply module body 11 shown in FIGS. 2 to 3 is used in terms of efficiency in dissolving hydrogen in the dialysate 27 by increasing an atmospheric pressure of the supplied hydrogen gas in a small amount.

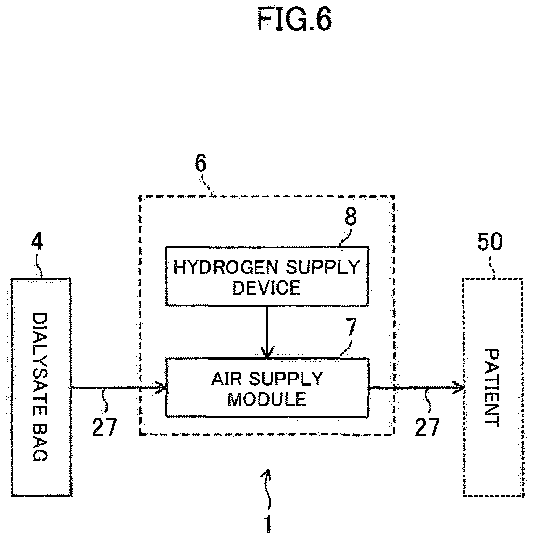

In the above embodiment, the dialysate 27 is supplied from the peritoneal dialysis apparatus 40 to the patient 50. However, as shown in FIG. 6, instead of using the peritoneal dialysis apparatus 40, the dialysate bag 4 may be connected to a tube (catheter) arranged in the patient 50, and the dialysate 27 housed in the dialysate bag 4 may be directly injected into the peritoneal cavity of the patient 50. In such a configuration, the same effect as that of the above embodiment can be obtained.

In this case, as shown in FIG. 6, the hydrogen dissolution device 6 is provided between the dialysate bag 4 and the patient 50, and the air supply module 7 (that is, the supply tube 10) in the hydrogen dissolution device 6 is connected to the dialysate bag 4 and the patient 50.

### INDUSTRIAL APPLICABILITY

As described above, the present invention is particularly useful for an apparatus for manufacturing hydrogen-containing peritoneal dialysate.

### DESCRIPTION OF REFERENCE CHARACTERS

- 1: Apparatus for Manufacturing Hydrogen-containing Peritoneal Dialysate
- 4: Dialysate Bag
- 6: Hydrogen Dissolution Device
- 7: Air Supply Module
- 8: Hydrogen Supply Device
- 10: Supply Tube
- 11: Air Supply Module Body
- 12: Hydrogen Supply Port
- 27: Dialysate
- 40: Peritoneal Dialysis Apparatus
- 50: Patient

## Claims

1. An apparatus (1) for manufacturing hydrogen-containing peritoneal dialysate, the apparatus (1) comprising:
a dialysate bag (4) housing dialysate (27) to be injected into a peritoneal cavity of a patient; and
a hydrogen dissolution device (6) interposed between the dialysate bag (4) and the patient and configured to dissolve hydrogen in the dialysate (27), wherein
the hydrogen dissolution device (6) includes: an air supply module (7) connected to the dialysate bag (4) and the patient and configured to dissolve hydrogen in the dialysate (27); and a hydrogen supply device (8) connected to the air supply module (7) and configured to supply hydrogen to the air supply module (7),
the air supply module (7) includes: a supply tube (10) connected to the dialysate bag (4) and the patient and configured to supply the dialysate (27) to the patient; and an air supply module body (11) that is connected to the hydrogen supply device (8) , houses the supply tube (10), and is configured to be supplied with the hydrogen,
with the dialysate (27) being supplied inside the supply tube (10), the hydrogen is supplied to the dialysate (27) by passing inside the air supply module body (11) and outside the supply tube (10), and **characterized in that**
the supply tube (10) is wound in a roll-like manner and housed in the air supply module body (11).

## Patentansprüche

1. Vorrichtung (1) zur Herstellung einer wasserstoffhaltigen Peritonealdialyseflüssigkeit, wobei die Vorrichtung (1) umfasst:
einen Dialyseflüssigkeitsbeutel (4), der eine Dialyseflüssigkeit (27) aufnimmt, die in eine Peritonealhöhle eines Patienten injiziert werden soll; und
ein Wasserstofflösungsgerät (6), das zwischen dem Dialyseflüssigkeitsbeutel (4) und dem Patienten eingeschoben ist und dazu konfiguriert ist, Wasserstoff in der Dialyseflüssigkeit (27) zu lösen, wobei
das Wasserstofflösungsgerät (6) beinhaltet: Ein Luftzufuhrmodul (7), das mit dem Dialyseflüssigkeitsbeutel (4) und dem Patienten verbunden ist und dazu konfiguriert ist, Wasserstoff in der Dialyseflüssigkeit (27) zu lösen; und ein Wasserstoffzufuhrgerät (8), das mit dem Luftzufuhrmodul (7) verbunden ist und dazu konfiguriert ist, dem Luftzufuhrmodul (7) Wasserstoff zuzuführen,
das Luftzufuhrmodul (7) beinhaltet: Einen Zufuhrschlauch (10), der mit dem Dialyseflüssigkeitsbeutel (4) und dem Patienten verbunden ist und dazu konfiguriert ist, dem Patienten die Dialyseflüssigkeit (27) zuzuführen; und ein Luftzufuhrmodulgehäuse (11), das mit dem Wasserstoffzufuhrgerät (8) verbunden ist, den Zufuhrschlauch (10) aufnimmt und dazu konfiguriert ist, dass ihm der Wasserstoff zugeführt wird,
der Wasserstoff bei der innerhalb des Zufuhrschlauchs (10) zugeführten Dialyseflüssigkeit (27) der Dialyseflüssigkeit (27) durch Leiten innerhalb des Luftzufuhrmodulgehäuses (11) und außerhalb des Zufuhrschlauchs (10) zugeführt wird, und **dadurch gekennzeichnet, dass**
der Zufuhrschlauch (10) auf eine rollenartige Weise gewickelt wird und in dem Luftzufuhrmodulgehäuse (11) aufgenommen wird.

## Revendications

1. Appareil (1) pour fabriquer un dialysat péritonéal contenant de l'hydrogène, l'appareil (1) comprenant :
une poche de dialysat (4) recevant le dialysat (27) à injecter dans une cavité péritonéale d'un patient ; et
un dispositif de dissolution d'hydrogène (6) interposé entre la poche de dialysat (4) et le patient et configuré pour dissoudre l'hydrogène dans le dialysat (27), dans lequel
le dispositif de dissolution d'hydrogène (6) comprend : un module d'alimentation en air (7) relié à la poche de dialysat (4) et au patient et configuré pour dissoudre l'hydrogène dans le dialysat (27) ; et un dispositif d'alimentation en hydrogène (8) relié au module d'alimentation en air (7) et configuré pour fournir de l'hydrogène au module d'alimentation en air (7),
le module d'alimentation en air (7) comprend : un tube d'alimentation (10) relié à la poche de dialysat (4) et au patient et configuré pour fournir le dialysat (27) au patient ; et un corps de module d'alimentation en air (11) qui est relié au dispositif d'alimentation en hydrogène (8), reçoit le tube d'alimentation (10), et est configuré pour être alimenté en hydrogène,
le dialysat (27) étant fourni à l'intérieur du tube d'alimentation (10), l'hydrogène est fourni au dialysat (27) en passant à l'intérieur du corps de module d'alimentation en air (11) et à l'extérieur du tube d'alimentation (10), et **caractérisé en ce que**
le tube d'alimentation (10) est enroulé sous forme de rouleau et reçu dans le corps de module d'alimentation en air (11).
